# EUROPÄISCHE PATENTANMELDUNG

(11) **EP 4 726 381 A1**
(43) Veröffentlichungstag der Anmeldung: **15.04.2026**
(21) Anmeldenummer: 25206813.5
(22) Anmeldetag: 06.10.2025
(51) Int. Cl.: G01N 25/48

(54) **SYSTEM ZUR SIMULTANEN THERMISCHEN ANALYSE EINER VIELZAHL VON EINZELPROBEN EINES, INSBESONDERE BIOLOGISCHEN, MATERIALS DURCH DYNAMISCHE DIFFERENZKALORIMETRIE (DSC), PROBENTRÄGER UND VERFAHREN ZUR SIMULTANEN ANALYSE EINER VIELZAHL VON EINZELPROBEN**

(30) Priorität: 09.10.2024 DE 102024129206
(71) Anmelder: Netzsch-Gerätebau GmbH, 95100 Selb (DE)
(72) Erfinder: Taubmann, Rebekka, 95100 Selb (DE); Rummel, Florian, 95100 Selb (DE); Küspert, Sabrina, 95100 Selb (DE); Hollering, Markus, 95632 Wunsiedel (DE); Dunkel, Johannes, 95111 Rehau (DE)

(57) **Zusammenfassung**

Die vorliegende Erfindung schafft ein System zur simultanen thermischen Analyse einer Vielzahl von Einzelproben eines, insbesondere biologischen, Materials durch dynamische Differenzkalorimetrie (DSC), mit wenigstens einem, mehrere Probengefäße aufweisenden Probenträger, wobei jedem Probengefäß ein Einzelsensor zur Messung einer von der Einzelprobe während der thermischen Analyse abgegebenen oder aufgenommenen Wärmemenge zugeordnet ist; einer Heiz- und/oder Kühleinheit zur simultanen Temperaturbeaufschlagung der in den Probengefäßen beinhalteten Einzelproben, mit einer Aufnahme für den wenigstens einen Probenträger; einer Messeinrichtung, welche mit den Einzelsensoren verbunden ist, und dazu ausgebildet ist einen Messwert für die abgegebene oder aufgenommene Wärmemenge der Einzelproben während der thermischen Analyse simultan zu erfassen, einen Probenträger, insbesondere zur Verwendung in diesem System sowie ein Verfahren zur simultanen Analyse einer Vielzahl von Einzelproben oder Gruppen von Einzelproben durch dynamische Differenzkalorimetrie (DSC).

## Beschreibung

Die vorliegende Erfindung betrifft ein System zur simultanen thermischen Analyse einer Vielzahl von Einzelproben eines, insbesondere biologischen, Materials durch dynamische Differenzkalorimetrie (DSC), einen Probenträger, insbesondere zur Verwendung in einem solchen System sowie ein Verfahren zur simultanen Analyse einer Vielzahl von Einzelproben.

Die dynamische Differenzkalorimetrie (differential scanning calorimetry - DSC) ist ein Verfahren der thermischen Analyse zur Messung von abgegebener oder aufgenommener Wärmemenge einer Probe bei Aufheizung, Abkühlung oder einem isothermen Prozess. Die dynamische Differenzkalorimetrie kann für eine Vielzahl von Analysen wie beispielsweise die Analyse von Schmelz- und Glasübergangstemperaturen, des Kristallisationsgrads, der kinetischen Betrachtungen chemischer Reaktionen, der spezifische Wärmekapazität und von Phasenübergängen eingesetzt werden. Auch erlaubt die dynamische Differenzkalorimetrie (DSC) Analysen zum Erkennen von Krankheiten und im medizinischen Bereich in der Forschung angewendet werden. Typischerweise erlauben Messapparaturen zur Durchführung von Analysen mittels dynamischer Differenzkalorimetrie lediglich die gleichzeitige Analyse von wenigen Proben oder Einzelproben. Bei einem hohen Probendurchsatz ist daher und aufgrund geringer Kapazitäten für Analysen pro Zeiteinheit insbesondere die Wartezeiten für benötigte Analysen sehr lange. Auch lassen sich Analysen nicht in einem wirtschaftlich Umfang durchführen da die Einzelprobenmessung mit einem entsprechend hohen Zeitaufwand einhergeht. Auch benötigen herkömmliche Messapparaturen zur Durchführung von reproduzierbaren Messungen große Probenvolumina von 15µl und mehr. Bekannte Messapparaturen ermöglichen im kommerziellen Bereich daher keinen wirtschaftlichen Einsatz.

Es sind Vorrichtungen und Verfahren bekannt, wie sie typischerweise zu Forschungszwecken Anwendung finden.

Die US 2019/0003995 A1 beschreibt eine dynamische Differenzkalorimeter-Vorrichtung zum Erkennen von Krankheiten und Überwachen der therapeutischen Wirksamkeit durch das Erkennen wärmebeständiger Varianten von Proteinen und/oder Stoffwechselprodukten in biologischen Proben.

Die WO 2017/066800 A1 beschreibt Verfahren zum Charakterisieren und/oder Vorhersagen von Risiko verbunden mit einer biologischen Probe unter Verwendung von thermischen Stabilitätsprofilen.

Es ist Aufgabe der vorliegenden Erfindung, Möglichkeiten zur kostengünstigeren, einfacheren und schnelleren Durchführung von Analysen mittels dynamischer Differenzkalorimetrie zu schaffen.

Erfindungsgemäß wird diese Aufgabe jeweils durch die Gegenstände der unabhängigen Ansprüche gelöst.

Gemäß einem ersten Aspekt der Erfindung ist ein System zur simultanen thermischen Analyse einer Vielzahl von Einzelproben eines, insbesondere biologischen, Materials durch dynamische Differenzkalorimetrie (DSC) vorgesehen. Das System umfasst dabei wenigstens einen, mehrere Probengefäße aufweisenden Probenträger, wobei jedem Probengefäß ein Einzelsensor zur Messung einer von der Einzelprobe während der thermischen Analyse abgegebenen oder aufgenommenen Wärmemenge zugeordnet ist, eine Heiz- und/oder Kühleinheit zur simultanen Temperaturbeaufschlagung der in den Probengefäßen beinhalteten Einzelproben, mit einer Aufnahme für den wenigstens einen Probenträger, eine Messeinrichtung, welche mit den Einzelsensoren verbunden ist, und dazu ausgebildet ist einen Messwert für die abgegebene oder aufgenommene Wärmemenge der Einzelproben während der thermischen Analyse simultan zu erfassen.

Gemäß einem zweiten Aspekt der Erfindung ist ein Probenträger, insbesondere zur Verwendung im vorgenannten System vorgesehen, wobei der Probenträger eine Vielzahl von bevorzugt in einem definierten Raster angeordneten Probengefäßen für jeweils eine Einzelprobe aufweist.

Gemäß einem dritten Aspekt der Erfindung ist ein Verfahren zur simultanen Analyse einer Vielzahl von Einzelproben oder Gruppen von Einzelproben eines insbesondere biologischen Materials durch dynamische Differenzkalorimetrie (DSC), insbesondere in einem vorgenannten System vorgesehen. Das Verfahren umfasst dabei die Schritte: Einbringen der Einzelproben in Probengefäße in einem Probenträger, insbesondere einen Probenträger wie zuvor beschrieben, mit dem jeweiligen Probegefäß zugeordneten Einzelsensoren, Einbringen des Probenträgers in eine Heiz- und/oder Kühleinheit, Verbinden der Einzelsensoren mit einer Messeinrichtung, Durchführen einer thermischen Analyse mit simultaner Messung von abgegebener oder aufgenommener Wärmemenge der Einzelproben während der thermischen Analyse durch die Einzelsensoren, simultanes Erfassen von Messwerten der Einzelproben oder Gruppen von Einzelproben durch die Messeinrichtung, Senden der Messwerte an eine Auswerteeinheit, die mit der Messeinrichtung kommuniziert und simultanes Auswerten der Messwerte und Ableitung charakterisierender Datenstrukturen der Einzelproben oder Gruppen von Einzelproben auf Basis der Messwerte mittels der Auswerteeinheit.

Gemäß einem vierten Aspekt der Erfindung ist die Verwendung eines erfindungsgemäßen Systems und/oder eines erfindungsgemäßen Probeträgers zur simultanen thermischen und/oder visuellen Analyse von insbesondere biologischen Einzelproben durch dynamische Differenzkalorimetrie (DSC) vorgesehen.

Eine der vorliegenden Erfindung zugrunde liegende Idee besteht darin, reproduzierbare Messungen auch kleiner Probenvolumina mit hohem Probendurchsatz bei gleichzeitig reduziertem Zeitaufwand für u.a. Probenvorbereitung und Analyse der Messergebnisse durchzuführen. Dabei kann die Erfindung sowohl die Produktentwicklungszyklen insbesondere, jedoch nicht hierauf beschränkt, in der biotechnologischen Entwicklung beschleunigen sowie in der insbesondere medizinischen Diagnostik Wartezeiten auf Analyseergebnisse verkürzen. Auch erlaubt die vorgeschlagene Erfindung die simultane Analyse mehrerer Proben unter gleichen Analysebedingungen und daraus die Ableitung von differenzierten Mess- und Analyseergebnissen.

Vorteilhafte Ausführungsformen und Weiterbildungen ergeben sich aus den auf die unabhängigen Ansprüche rückbezogenen Unteransprüchen sowie aus der Beschreibung unter Bezugnahme auf die Figuren.

Gemäß einer Ausführungsform des Systems ist die Aufnahme in einer Prüfkammer angeordnet, wobei die Umgebungsbedingungen innerhalb der Prüfkammer definiert oder variabel regelbar ausgebildet sind. Somit kann die simultane Analyse mehrerer Proben in einem Messzyklus und unter gleichen Umgebungs- bzw. Messbedingungen bzw. unter gleicher Variation der Umweltbedingungen, beispielsweise im Verlauf einer Analyse durchgeführt werden. Dies bietet Vorteile beim Probendurchsatz aber auch bezüglich der Reproduzierbarkeit der Messergebnisse und erlaubt die Durchführung von simultanen Analysen mehrerer gleichartiger Probenserien unter identischen Bedingungen. In diesem Zusammenhang sind die definierten oder variabel regelbaren Umgebungsbedingungen innerhalb der Prüfkammer insbesondere ausgewählt aus: Temperatur, Druck, relative Luftfeuchtigkeit, (Inert-) Gasatmosphäre und Kombinationen daraus.

Gemäß einer Weiterbildung ist im erfindungsgemäßen System ferner eine Auswerteeinheit vorgesehen, welche dazu ausgebildet ist, die Messwerte von der Messeinrichtung zu empfangen, zu analysieren und charakterisierende Datenstrukturen auf Basis der Messwerte für jede Einzelprobe abzuschätzen. Das System erlaubt es so, simultan Datenstrukturen aus Messwerte von Einzelproben abzuleiten, die unter identischen Bedingungen erfasst wurde und somit hinsichtlich aller Parameter vollumfänglich vergleichbar sind. Das Erzielen einer Reproduzierbarkeit über eine großen Anzahl von Messungen und Proben hinweg wird somit möglich und verbessert.

Gemäß einer weiteren Weiterbildung ist im System ein Display vorgesehen, welches dazu ausgebildet ist, Messwerte und/oder Datenstrukturen zu visualisieren. Die unmittelbare Darstellung des Analyseverlaufs und der erfassten Messwerte und/oder Datenstrukturen ist somit wesentlich vereinfacht und erlaubt dem Nutzer des System eine Echtzeitüberwachung der Analyse und der erfassten Messwerte vor oder nach einer Auswertung und die direkte Darstellung sowie den Vergleich der Messwerte und Analyseergebnisse. Dies biete auch unter Qualitätsüberwachungsgesichtspunkten Vorteile und kann zur Effizienzsteigerung bei der Systemnutzung beitragen. Das Display kann gleichzeitig zur Darstellung der Analyseparameter genutzt werden. Ist das Display beispielsweise als Touchscreen ausgebildet, dient es gleichzeitig zur Systemsteuerung und zur Eingabe bzw. Auswahl von Analyseparametern oder Analyseprogrammen des Systems.

Gemäß einer weiteren Ausführungsform des Systems sind die Einzelsensoren in einer Sensorplatte zusammengefasst. Die Anzahl der Einzelsensoren der Sensorplatte entspricht dabei der Anzahl der Probenaufnahmegefäßen im Probenträger. Die Einzelsensoren sind dabei an Positionen der Probengefäße entsprechenden Positionen in der Sensorplatte angeordnet. Die Verwendung einer Sensorplatte trägt zur Vereinfachung des Systems und zur verbesserten Positionierung der in der Sensorplatte zusammengefassten und an definierten, standardisierbaren Positionen in der Sensorplatte festgelegten Sensoren relativ zur Probe bzw. zum Probengefäß bei. Dadurch wird auch eine gleichbleibend hohe Qualität der Messergebnisse sichergestellt, da die Positioniergenauigkeit und Positionstreue der Sensoren gewährleistet ist.

In diesem Zusammenhang sieht eine weitere vorteilhafte Ausführungsform des erfindungsgemäßen Systems vor, dass die Sensorplatte als gesondertes, mit dem Probenträger form- oder kraftschlüssig verbindbares Element zur Verfügung gestellt ist. Die Sensorplatte kann so in einfacher Art und Weise mit dem Probenträger verbunden, beispielsweise auf diesen aufgesteckt, mit diesem verrastet oder mit dem Probenträger mittels einer Clipsverbindung oder dergleichen verbunden werden. Die Messung bzw. die die Messung vorbereitenden Maßnahmen wie beispielsweise Probenvorbereitung oder Vorbereitung der Probenträger wird dadurch wesentlich beschleunigt und die Positionierung verbessert und reproduzierbar. Auch können die mit den Sensoren verbundenen Datenleitungen in der Sensorplatte zusammengefasst werden und über eine Verbindung, beispielsweise Steckverbindung in das System eingebunden werden.

Gemäß einer weiteren alternativen Ausführungsform des Systems ist vorgesehen, dass die Sensorplatte als dauerhaft in der Aufnahme angeordnetes Element ausgebildet ist und dort verbleibt. Zur Durchführung der Messung muss der Probenträger lediglich auf die bevorzugt ortsfest in der Aufnahme festgelegte Sensorplatte aufgesetzt, aufgesteckt oder mit dieser verrastet bzw. verclipst werden. Dadurch dass die Sensorplatte dauerhalft in der Aufnahme und damit im System verbleibt, ist die Positionierung des Probenträgers wesentlich einfacher und schneller zu bewerkstelligen. Da die Sensorplatte nicht unmittelbar mit der Probe in Kontakt kommt, besteht keine Gefahr von Kontaminationen oder Kreuzkontaminationen innerhalb des Systems. Auch besteht keine Notwendigkeit, die Sensorplatte nach der Messung zu reinigen oder aufwändig zu sterilisieren, wodurch die Benutzerfreundlichkeit des System erhöht und der Probendurchsatz pro Zeiteinheit erhöht werden kann.

Gemäß einer weiteren, alternativen Ausführungsform des erfindungsgemäßen Systems ist ein in jedes Probengefäß integrierter oder ein mit jedem Probengefäß form- oder kraftschlüssig verbindbarer Einzelsensor vorgesehen. Hierdurch wird eine individuelle Ansteuerung jedes einzelnen Probengefäßes möglich. Auch ermöglicht diese Ausführungsform, dass nur teilweise befüllte Probenträger ausgewertet werden können.

Gemäß einer weiteren Ausführungsform entspricht eine Grundfläche des Einzelsensors im Wesentlichen einer Grundfläche des Probengefäßes. Dies stellt sicher, dass Messwerte über die gesamte Grundfläche des Probengefäßes erfasst und die Messgenauigkeit somit wesentlich erhöht wird.

Gemäß einer weiteren Ausführungsform umfasst die Auswerteeinheit ferner eine Schnittstelle bzw. Kommunikationsschnittstelle, welche dazu ausgebildet ist, eine Kommunikationsverbindung zwischen der Auswerteeinheit und einem externen Kommunikationsteilnehmer aufzubauen. Die ermöglicht es, dass die Auswerteeinheit aus dem System Datensätze, insbesondere auf Datenspeicher, wie eine Cloud oder ähnliches, oder auf Server übermittelt ohne selbst mit einem Server oder dergleichen kommunikativ gekoppelt zu werden. Auf diese Weise können die Daten leichter übertragen und erforderlichenfalls während der Übertragung geschützt werden. Die Schnittstelle kann dabei als Schnittstelle zur kabelgebundenen oder kabellosen Datenübertragung ausgebildet werden. Eine Übertragung beispielsweise per Bluetooth, eine WLAN-Verbindung oder mit einem anderen dem Fachmann geläufigen Kommunikationsstandard ist dabei möglich und von der Erfindung umfasst.

Gemäß einer Weiterbildung umfasst das System ferner eine Beleuchtungseinheit, wobei die Beleuchtungseinheit ausgewählt ist aus: einer UV-Licht-Beleuchtungseinheit, einer Beleuchtungseinheit, welche ausgelegt ist, sichtbares Licht zu emittieren, einer Beleuchtungseinheit, welche ausgelegt ist, infrarotes Licht zu emittieren, einer Beleuchtungseinheit, welche ausgelegt ist, polarisiertes Licht zu emittieren, einer Beleuchtungseinheit, welche ausgelegt ist, fluoreszierendes Licht, insbesondere blaues, grünes oder rotes fluoreszierendes Licht, zu emittieren. Das System ermöglicht damit eine gleichzeitige Spektralanalyse der Proben während der thermischen Analyse und erweitert das mit dem System erfassbare Mess- und Datenspektrum. Auch können Prozesse innerhalb der Probe während der thermischen Analyse visuell erfasst bzw. sichtbar gemacht werden.

Gemäß einer weiteren Ausführungsform des Systems sind die Heiz- und/oder Kühleinheit, die Aufnahme für den wenigstens einen Probenträger, die Messeinrichtung, die Auswerteeinheit, die Prüfkammer, die Beleuchtungseinheit und/oder das Display von einem Gehäuse, insbesondere einem gemeinsamen Gehäuse, zumindest teilweise umgeben. Somit sind das System oder zumindest Komponenten des Systems kompakt in einer Einheit zusammengefasst und es wird auch die Ausbildung eines mobil tragbaren Systems ermöglicht.

Gemäß einer weiteren Ausführungsform des Systems ist eine Schnittstelle und/oder ein Sichtfenster zur Anordnung einer optischen Auswertvorrichtung, insbesondere eine Kameravorrichtung, eine Mikroskopvorrichtung, ein Fluoreszenzmikroskop oder eine Raman-Vorrichtung vorgesehen. Hierdurch wird eine visuelle Inspektion des Ablaufs des Analyseverfahrens und eine optische Analyse der Proben ermöglicht und die Auswertung der Messergebnisse somit verbessert bzw. die Bandbreite der mit dem System durchführbaren Analyseverfahren erweitert, dies insbesondere im Zusammenhang und unter Verwendung der zuvor beschriebenen Beleuchtungseinheit und den dadurch zur Verfügung gestellten Beleuchtungsoptionen.

Gemäß einer weiteren Ausführungsform des Systems ist die Schnittstelle und/oder das Sichtfenster in das Gehäuse und/oder die Prüfkammer integriert vorgesehen. Die Integration der Schnittstelle ermöglicht die einfache und schnelle Verbindung des Systems mit Drittgeräten und die Anbindung optischer Auswertvorrichtungen, während das Sichtfenster unmittelbaren optischen Zugriff auf die Proben ermöglicht und die visuelle Auswertung beispielsweise mittels Kameraeinrichtung oder Mikroskop zulässt.

Gemäß einer weiteren Ausführungsform ist der Probenträger als Mikrotiterplatte mit standardisierter Konfiguration und die Aufnahme als Einsetzfach für die Mikrotiterplatte ausgebildet. Auf diese Weise kann ein hoher Probendurchsatz ermöglicht werden, während die Mikrotiterplatte, abhängig von ihrer jeweiligen Konfiguration, auch die Messung kleiner Volumina von insbesondere weniger als 10 µl Probenvolumen in der Einzelprobe erlaubt. Die Benutzerfreundlichkeit des Systems wird durch das Einsetzfach wesentlich verbessert, da vor jeder Messung eine reproduzierbare Positionierung der Mikrotiterplatte im System vorgenommen kann.

Gemäß einer Weiterbildung des Probenträgers ist dieser als eine aus einem temperaturbeständigen Material, insbesondere Kunststoffmaterial gefertigte standardisierte Mikrotiterplatte mit zwischen 6 und 1546 Probenaufnahmegefäßen ausgebildet. Auf diese Weise wird die simultane Messung einer Vielzahl von Proben mit hoher Reproduzierbarkeit ermöglicht und der Probendurchsatz dadurch wesentlich erhöht. Zudem erlaubt die Verwendung standardisierte Mikrotiterplatten, beispielsweise im labortypischen, sog. 96er Mikrotiterplattenformat, die ebenfalls standardisierte Ausführung der zuvor beschriebenen Sensorplatte zur Verbindung mit dieser. Eine positionsgenaue Anordnung der Messsensoren relativ zu den Probengefäßen in der Mikrotiterplatte wird dadurch vereinfacht. Die Probenvorbereitung, die aufgrund des Standardformats des Probenträgers auch automatisiert durchgeführt werden kann wird ebenfalls wesentlich beschleunigt, mit entsprechenden Vorteilen für den Probendurchsatz. Die standardisierte Mikrotiterplatte kann zudem in entsprechend abgestimmte Aufnahmen im Messsystem eingesetzt werden und das System somit schnell und in einfacher Art und Weise mit einer Vielzahl von Proben bestückt werden. Nach Abschluss der Analyse kann die Mikrotiterplatte verworfen oder gereinigt und wiederverwendet werden, wobei das System sofort wieder für die nächste Messung zur Verfügung steht.

Gemäß einer weiteren Ausführungsform des Probenträgers weist das Kunststoffmaterial eine Temperaturbeständigkeit in einem Temperaturbereich von zwischen -200 °C und +250 °C, bevorzugt von zwischen -80 °C und +200 °C auf. Dies erlaubt eine Messung in einem hohen Temperaturbereich, ohne dass eine Beeinträchtigung des Probenträgers während der Messung stattfindet. Des Weiteren besteht auch die Möglichkeit, dass direkt aus einer Tieftemperaturlagerung kommende Probenträger für die Messung verwendet werden können. Dadurch kann eine Vielzahl von Proben für die Messung vorbereitet, bei Tiefsttemperaturen, beispielsweise -80 °C, gelagert und dann in im System abgearbeitet werden, ohne dass weitere Vorbereitungshandlungen vor der jeweiligen Analyse bzw. Messung notwendig sind. Die Effizienz des Systems und der Probendurchsatz wird dadurch erhöht.

Gemäß einer weiteren Ausführungsform des Probenträgers ist vorgesehen, dass jedem Probengefäß ein Einzelsensor zur Messung von durch die Einzelproben abgegebener oder aufgenommener Wärmemenge während einer thermischen Analyse zugeordnet ist. Das System arbeitet somit mit einer Vielzahl von Einzelsensoren, die die Messungen simultan vornehmen. Durch die klare Zuordnung des Einzelsensors zu einem Probengefäß ist sichergestellt, dass durch den Sensor ausschließlich die jeweilige Einzelprobe gemessen wird. Die Einzelsensoren sind dabei hinreichend miniaturisiert, sodass Messwerte aus Einzelproben in den zuvor beschriebenen Mikrotiterplatte auch in kleinsten Messvolumina zuverlässig und reproduzierbar erfasst werden können.

Gemäß einer weiteren Ausführungsform des Probenträgers ist vorgesehen, dass der jeweilige Einzelsensor in das Probengefäß integriert oder mit dem Probengefäß form- oder kraftschlüssig verbindbar ist. Auf diese Weise kann ein Probenträger zur Verfügung gestellt werden, bei dem in jedem oder auch nur in einzelnen Probengefäßen jeweils ein Einzelsensor angeordnet ist. Es wird somit ein für die Messung im vorher beschriebenen System geeigneter und entsprechend vorbereiteter Probenträger zur Verfügung gestellt. Dieser kann als Verbrauchsartikel oder als Zubehörteil für das System zur Verfügung gestellt werden.

In einer hierzu alternativen Ausführungsform besteht die Möglichkeit, dass der jeweilige Einzelsensor mit dem jeweiligen Probengefäß bedarfsweise verbunden wird. Dies erlaubt die Verwendung standardisierter Probengefäße, die für die Messung durch Bestückung mit Einzelsensoren vorbereitet werden können. Die Probengefäße, beispielsweise standardisierte Mikrotiterplatte können nach der Verwendung vom Einzelsensor getrennt, entsorgt oder gereinigt und wieder verwendet werden. Hierdurch kann die Flexibilität des Systems aber auch die Effizienz bei der Durchführung von Messungen und damit der Probendurchsatz erhöht werden.

Gemäß einer weiteren Ausführungsform ist vorgesehen, dass die Einzelsensoren in einer Sensorplatte zusammengefasst sind und die Anzahl der Einzelsensoren der Sensorplatte der Anzahl der Probenaufnahmegefäßen im Probenträger entspricht. Die Einzelsensoren sind dabei an Positionen der Probengefäße entsprechenden Positionen in der Sensorplatte angeordnet. Die Verwendung einer Sensorplatte vereinfacht die Handhabung des Systems wesentlich, da die Sensorplatte, die in ihrer Grundfläche und Anordnung der Sensoren der Konfiguration des Probenträgers und der dort vorgesehenen Anordnung der Probengefäße entspricht, vor der Messung mit dem Probenträger verbunden werden kann. Die Verbindung kann dabei beispielsweise durch Aufstecken, Verrasten, Anclipsen oder in sonstiger geeigneter Art und Weise zur Ausbildung einer form- oder kraftschlüssigen Verbindung erfolgen. Die Verbindung zwischen Sensorplatte und Probenträger weist dabei eine hohe Positionsgenauigkeit auf, sodass sichergestellt ist, dass die in den Probengefäßen beinhalteten Proben reproduzierbar und mit hoher Genauigkeit gemessen werden. Nach erfolgter Analyse kann die Sensorplatte in einfacher Art und Weise vom Probenträger getrennt und für die nächste Messung bereitgestellt werden. Es wird somit ein modulares System zur Verfügung gestellt, dass auch hocheffizient und zur Abarbeitung eines hohen Probendurchsatzes verwendet werden kann, wodurch Zeit- und Kostenersparnisse realisiert werden können.

Gemäß einer Ausführungsform des Verfahrens wird die Analyse unter definierten oder variabel regelbaren Umgebungsbedingungen durchgeführt. Die Umgebungsbedingungen sind dabei ausgewählt aus Temperatur, Druck, relative Luftfeuchtigkeit, (Inert-) Gasatmosphäre und Kombinationen daraus. Im Verfahren ist dabei vorgesehen, dass die Umgebungsbedingungen während der Messung durch das System gleich gehalten werden, oder aber das entsprechende Profile für die Umgebungsbedingungen, d.h. beispielsweise eine variable Temperatur, variabler Druck, eine Variation der Luftfeuchtigkeit oder der Gasatmosphäre im System erzeugt werden, wodurch das Verhalten der Proben unter variierenden Parametern der Umweltbedingungen gemessen werden können. Die jeweiligen Umweltbedingungen im System werden durch geeignete Sensoren, die beispielsweise in der Prüfkammer oder dem wie zuvor beschriebenen Gehäuse, angeordnet sind, überwacht. Die Umgebungsbedingungen können dabei ebenfalls auf dem zuvor beschriebenen Display angezeigt, oder in die Datensätze integriert ausgegeben werden, um eine vollständige Nachvollziehbarkeit und/oder Reproduzierbarkeit der Messung zu gewährleisten.

Gemäß einer Weiterbildung des Verfahrens, umfasst dieses ferner eine visuelle Analyse der Einzelproben oder Gruppen von Einzelproben mittels einer optischen Auswertvorrichtung, insbesondere einer Kameravorrichtung, einer Mikroskopvorrichtung, einem Fluoreszenzmikroskop oder einer Raman-Vorrichtung. Ergänzend oder alternativ zu der Erfassung der Messwerte über die zuvor beschriebenen Sensoren, kann somit auch eine visuelle Auswertung der Proben während des Analysevorgangs erfolgen, die zusätzliche Rückschlüsse über die Zusammensetzung der Probe oder das Verhalten der beprobten Materialien während der thermischen Analyse zulässt. Die visuelle Inspektion der Einzelproben oder Gruppen von Einzelproben erfolgt dabei bevorzugt über die zuvor beschriebene Schnittstelle oder ein entsprechendes Sichtfenster in der Prüfkammer oder dem Gehäuse des Systems. Auch besteht die Möglichkeit, dass die Erfassung visueller Messwerte automatisiert im System erfolgt und entsprechende Datensätze mit den durch die Sensoren erfassten Datensätze kombiniert durch das System ausgegeben werden. Die Kombination aus erfassten Sensorwerten und zusätzlich oder alternativ erfassten visuellen Parametern der Proben erhöht die Flexibilität und damit den Einsatzbereich des Systems, dass somit für verschiedene Messaufgaben in einfacher Art und Weise angepasst oder für diese bereitgestellt werden kann.

In einer Weiterbildung des Verfahrens ist vorgesehen, dass die Analyse in einem definierten Temperaturprofil durchgeführt wird. Über die zuvor beschriebene Variationsmöglichkeit der Umgebungsbedingungen kann dies in einfacher Art und Weise sowie reproduzierbar durchgeführt und automatisch oder manuell gesteuert werden.

In einer bevorzugten Ausführungsform des erfindungsgemäßen Verfahrens ist vorgesehen, dass ein simultanes Auswerten der Einzelproben in der Auswerteeinheit automatisiert oder manuell durchgeführt wird. Dadurch, dass eine Vielzahl von Einzelproben im erfindungsgemäßen System gleichzeitig thermisch analysiert wird, kann der Probendurchsatz wesentlich erhöht und die Effizienz des Systems verbessert werden. Es kann somit eine Vielzahl von Datensätzen zur Verfügung gestellt werden, die bei automatisierter Auswertung unmittelbare Rückschlüsse auf das Probenverhalten, bzw. die Zusammensetzung der Proben erlaubt. Vergleiche zwischen unterschiedlichen Proben werden somit in einem Mess- oder Analysedurchgang ermöglicht. Die Möglichkeit zur manuellen Auswertung erlaubt es dem Nutzer, Parameter während der Analyse bzw. für den nachfolgenden Analysevorgang anzupassen. Im System wird hierzu eine entsprechende Software zur Verfügung gestellt, die alle parallel durchgeführten Messungen aufzeichnet und simultan auswertet. Die Software kann dabei mit einer integrierten Datenbank verbunden werden, sodass komplett automatisierte Auswertungen möglich sind. Parallel hierzu oder als Alternative ist auch die manuelle Auswertung möglich. Während der Messung kann jede Probe zusätzlich oder alternativ optisch analysiert werden. Die entsprechenden Messwerte werden dabei ebenfalls automatisiert oder manuell erfasst und bevorzugt softwareunterstützt ausgewertet.

In einer Weiterbildung umfasst das Verfahren ferner den Schritt des Visualisierens der Datenstrukturen, insbesondere auf einem Display. Auf diese Art und Weise kann die Analyse in Echtzeit verfolgt werden, wobei die erfassten Messwerte zusätzlich über die zuvor beschriebene Schnittstelle an eine nachfolgende Auswerteinstanz weitergegeben werden. Durch die Visualisierung der Datenstrukturen auf einem Display können zudem Messfehler oder Fehlfunktionen des Systems schnell festgestellt werden.

Die obigen Ausführungsformen und Weiterbildungen lassen sich, sofern sinnvoll, beliebig miteinander kombinieren. Weitere mögliche Ausführungsformen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird dabei der Fachmann auch Einzelaspekte als Verbesserungen oder Ergänzungen zu der jeweiligen Grundform der vorliegenden Erfindung hinzufügen.

Die vorliegende Erfindung wird nachfolgend anhand von Ausführungsbeispielen unter Bezugnahme auf die beiliegenden Figuren der Zeichnungen näher erläutert. Von den Figuren zeigen:
- Fig. 1: eine schematische Darstellung eines Probenträgers gemäß eines Ausführungsbeispiels der Erfindung in der Draufsicht;
- Fig. 2: eine schematische Darstellung einer weiteren Ausführungsform des Probenträgers gemäß eines weiteren Ausführungsbeispiels der Erfindung in Seitenansicht;
- Fig. 3: eine schematische Darstellung eines Systems gemäß eines Ausführungsbeispiels der Erfindung in perspektivischer Darstellung; und
- Fig. 4: ein Ablaufdiagramm eines Verfahrens zur Analyse, insbesondere zur Analyse durch dynamische Differenzkalorimetrie (DSC), von biologischem Material, gemäß einem Ausführungsbeispiel der Erfindung.

In den Figuren der Zeichnung sind gleiche, funktionsgleiche und gleich wirkende Elemente, Merkmale und Komponenten - sofern nichts anderes ausgeführt ist - jeweils mit denselben Bezugszeichen versehen.

Obwohl vorliegend spezifische Ausführungsformen und Weiterbildungen dargestellt und beschrieben sind, wird der Fachmann bevorzugen, dass eine Vielzahl von alternativen und/oder gleichartigen Ausführungen die dargestellten und beschriebenen spezifischen Ausführungsbeispiele ersetzen können, ohne vom Umfang der vorliegenden Erfindung abzukehren. Diese Anmeldung soll allgemein alle Abwandlungen oder Änderungen der hierin beschriebenen spezifischen Ausführungsbeispiele abdecken.

Die beiliegenden Figuren sollen ein weiteres Verständnis von Ausführungsformen der Erfindung vermitteln und dienen im Zusammenhang mit der Beschreibung der Erklärung von Prinzipien und Konzepten der Erfindung. Andere Ausführungsbeispiele und viele der genannten Vorteile ergeben sich im Hinblick auf die Zeichnungen. Die Zeichnungen sind lediglich als schematische Zeichnungen zu verstehen und die Elemente der Zeichnungen sind nicht notwendigerweise maßstabsgetreu zueinander dargestellt. Richtungsangebende Terminologie wie etwa "oben", "unten", "links", "rechts", "über", "unter", "horizontal", "vertikal", "vorne", "hinten" und ähnliche Angaben werden lediglich zu erläuternden Zwecken verwendet und dienen nicht der Beschränkung der Allgemeinheit auf spezifische Ausgestaltungen wie in den Figuren gezeigt.

Gestrichelte Linien in den Figuren der Zeichnungen verdeutlichen, dass die Verbindungen zwischen den die gestrichelten Linien verbindenden Komponenten nicht zwingend physischen Kontakt miteinander haben müssen, sondern gleichermaßen drahtlos zueinander gekoppelt sein können.

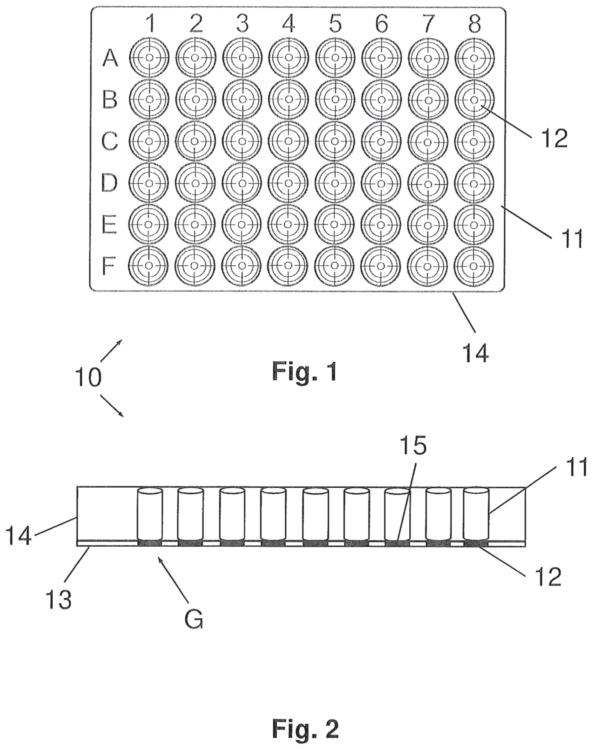

Fig. 1 zeigt eine schematische Darstellung eines Probenträgers 10 gemäß eines Ausführungsbeispiels der Erfindung in Draufsicht. Beim hier dargestellten Probenträger 10 handelt es sich um eine Mikrotiterplatte 14 mit insgesamt 56 einzelnen Probengefäßen 11, die in einem standardisierten Raster in der Mikrotiterplatte 14 angeordnet sind. Der Probenträger 10 ist selbstverständlich nicht auf die hier dargestellte Ausführungsformen und Konfiguration festgelegt. Gleichermaßen können im erfindungsgemäßen System 20 auch Mikrotiterplatten 14 mit weniger oder mehr Probengefäßen 11 verwendet werden. Das erfindungsgemäße System 20 ist auf die Verwendung von Probenträgern 10 mit insbesondere zwischen 6 und 1536 Probengefäßen 11 ausgelegt und erlaubt hier eine einzelprobenweise definierte und reproduzierbare Messung. Der erfindungsgemäße Probenträger 10 erlaubt die Messung von kleinen Volumina d.h. von Proben mit einem Probenvolumen von 10 µl oder weniger.

Im erfindungsgemäßen System 20 können somit standardisierte, im Laborumfeld erhältliche Mikrotiterplatten 14 verwendet werden. In der Ausführungsform gemäß Figur 1 weist der Probenträger 10 in die jeweiligen Probengefäße 11 integrierte Einzelsensoren 12 auf. Die Einzelsensoren 12 messen die abgegebene oder aufgenommene Wärmemenge der Einzelproben während der thermischen Analyse mittels dynamischer Differenzkalorimetrie (DSC). Die in Fig. 1 dargestellte Ausführungsform zeigt einen integrierten Probenträger 10, d.h. die Einzelsensoren 12 und der Probenträger 10 bzw. die Probengefäße 11 sind fest miteinander verbunden und die Einzelsensoren 12 in die Probengefäße 11 integriert. In einer alternativen, hier nicht dargestellten Ausführungsform besteht auch die Möglichkeit, dass die Einzelsensoren 12, in Form von Sensorplättchen, die die gleiche Grundfläche wie die Probengefäße 11 aufweisen, einzeln mit den jeweiligen Probengefäßen 11 lösbar verbunden, beispielsweise auf diesen aufgesteckt, an diese angeclipst oder in sonstiger Art und Weise mit diesen verbunden werden. Die Nutzung von Einzelsensoren 12 ermöglicht somit die individuelle, d.h. bedarfsweise Konfiguration der Probenträger 10.

Fig. 2 zeigt eine schematische Darstellung einer weiteren Ausführungsform des Probenträgers 10 gemäß eines weiteren Ausführungsbeispiels der Erfindung in Seitenansicht. Die Einzelsensoren 12, die bereits im Zusammenhang mit Fig. 1 beschrieben wurden, sind hier in einer unterhalb der Mikrotiterplatte 14 angeordneten Sensorplatte 13 zusammengefasst. Diese Sensorplatte 13 weist eine den Probengefäßen 11 im Probenträger 10 entsprechende Anzahl von Einzelsensoren 12 auf, die fest in eine Einheit, der Sensorplatte 13, gefügt sind.

Vor Durchführung einer thermischen Analyse mittels dynamischer Differenzkalorimetrie (DSC) wird diese Sensorplatte 13 mit dem Probenträger 10, im Ausführungsbeispiel einer Mikrotiterplatte 14 verbunden und verbleibt während der Messung in dieser Position. Die Verbindung mit dem Probenträger 10 erfolgt dabei lösbar, d.h. die Sensorplatte 13 wird auf den Probenträger 10 aufgesteckt, mit diesem verrastet oder an diesen angeclipst. Die Sensorplatte 13 ist dabei derart konfiguriert, dass eine positionsgenaue Anordnung an der Mikrotiterplatte 14 möglich ist. Nachdem die Sensorplatte 13 am Probenträger 10 angeordnet ist, befindet sich jeweils ein Einzelsensor 12 unterhalb eines jeden Probengefäßes 11 und deckt dessen gesamte Grundfläche G ab. Somit erfolgt eine vollumfängliche Messung über das gesamte Probengefäße 11 hinweg. Nach Abschluss der Analyse wird die Sensorplatte 13 vom Probenträger 10 abgenommen und kann sofort wieder an einen weiteren, für die nachfolgende Messung bestimmten Probenträger 10 angebunden werden. Dadurch, dass die Sensorplatte 13 nicht mit den zu messenden Proben in Berührung kommt, sondern eine Messung durch den Probenträger 10 bzw. den Boden 15 des Probenträgers 10 hindurch erfolgt, besteht keine Gefahr einer Kontamination der Sensorplatte 13, sodass eine Reinigung vor Einsatz für die nachfolgende Messung unterbleiben kann.

Gleichwohl kann die Sensorplatte 13 aus einem entsprechend sterilisierbaren Material gefertigt werden, wobei die Einzelsensoren 12 flüssigkeits- und gasdicht in der Sensorplatte 13 eingebettet sind. Bezüglich der geometrischen Dimension wie auch der Anzahl der Einzelsensoren 12 in der Sensorplatte 13 kann diese an verschiedene Konfigurationen von Probenträgern 10 angepasst werden, sodass stets eine passende Sensorplatte 13 für den jeweiligen Probenträger 10 zur Verfügung gestellt wird. Beim Probenträger 10 kann es sich beispielsweise um eine im Laborumfeld verwendbare Mikrotiterplatte 14 handeln, die zwischen 6 und 1536 Probengefäße 11, sogenannte wells, aufweist. Die Einzelsensoren 12 sind dabei auf die jeweils vorhandene Grundfläche der einzelnen Probengefäße 11 abgestimmt. Die Einzelsensoren 12 sind dabei hinreichend miniaturisiert, um eine Flächenüberdeckung der Probengefäße 11 bzw. des Bodens 15 der Probengefäße 11 zu gewährleisten, ohne dabei durch benachbarte Probengefäße 11 in der Messung beeinflusst zu werden. In der Sensorplatte 13 sind auch die jeweiligen Datenleitungen (nicht dargestellt) zu den Einzelsensoren 12 zusammengefasst, sodass die Sensorplatte 13 eine einzige Schnittstelle aufweist, die mit dem System 20 verbunden wird, um die erfassten Messwerte aus dem System 20 auszuleiten und einer Auswerteeinheit 16 zur Verfügung zu stellen. Für die thermische Analyse wird die Sensorplatte 13 Probenträger 10 befestigt und die Gesamteinheit bestehend aus Probenträger 10 und Sensorplatte 13 dann in das System 20 eingesetzt. Alternativ hierzu besteht auch die Möglichkeit, dass die Sensorplatte 13 bereits im Analysesystem installiert ist und lediglich der Probenträger 10 mit den darin aufgenommenen, zu messenden Einzelproben in eine dort befindliche Aufnahme eingesetzt und dabei oder danach erst mit der Sensorplatte 13 verbunden wird.

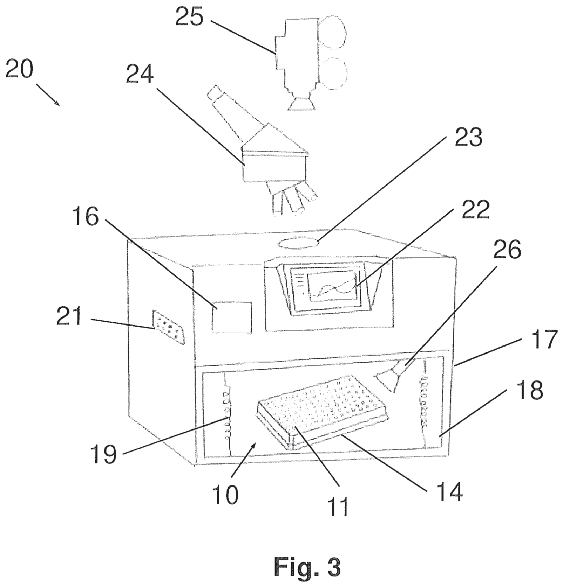

Fig. 3 zeigt eine schematische Darstellung eines Systems 20 gemäß einem Ausführungsbeispiel der Erfindung in perspektivischer Darstellung. Das erfindungsgemäße System 20 umfasst dabei ein Gehäuse 17, das eine Prüfkammer 18 beinhaltet, in die der Probenträger 10, der mit den zu analysieren Proben befüllt ist, eingestellt wird. Der Prüfkammer 18 zugeordnet befindet sich im Gehäuse 17 eine Heiz- bzw. Kühleinheit 19, über die eine
Temperaturbeaufschlagung der Proben anhand definierter Temperaturprofile durchgeführt werden kann. In der Prüfkammer 18 befindet sich eine hier nicht dargestellte Aufnahme für den Einsatz des Probenträgers 10. Beim Probenträger 10 handelt im Ausführungsbeispiel der Fig. 3 um eine entsprechend konfigurierte Mikrotiterplatte 14 mit einer Vielzahl von Probengefäßen 11, die fest in der Mikrotiterplatte 14 zusammengefasst sind. Diese Probengefäße 11 werden vor der thermischen Analyse mit den jeweiligen Proben befüllt und dann im erfindungsgemäßen System einer simultanen thermischen Analyse unterzogen. Zu diesem Zweck befindet sich unterhalb der des Probenträgers 10 angeordnet eine Sensorplatte 13, wie diese im Zusammenhang mit Fig. 2 bereits beschrieben wurde. Die Einzelsensoren 12 sind den jeweiligen Proben zugeordnet und erfassen dort Temperaturänderungen in der jeweiligen Probe während der thermischen Analyse. Die Sensordaten werden im System 20 unmittelbar ausgewertet. Hierzu umfasst das System 20 eine Auswerteeinheit 16, an die Sensordaten übermittelt und zur Auswertung bereitgestellt werden. Gleichzeitig besteht die Möglichkeit über die im System 20 vorhandene und am Gehäuse 17 angeordnete Schnittstelle 21 eine Ausleitung der Analysedaten zu einer nachgeordneten Auswertungsinstanz (nicht dargestellt) durchzuführen. Dabei kann es sich beispielsweise um eine Rechnereinheit mit entsprechender Auswertesoftware handeln. Durch die im Ausführungsbeispiel dargestellte Ausführungsform des Systems 20 kann mittels der Auswerteeinheit 16 auch eine unmittelbare Auswertung der erfassten Werte erfolgen. Die ausgewerteten Ergebnisse werden dann auf dem im Gehäuse 17 angeordneten Display 22 visuell dargestellt. Das Display 22 dient auch dazu die Betriebsparameter des Systems 20 darzustellen, beispielsweise eine Temperaturänderung, ein Temperaturgradient oder sonstige Umgebungsbedingungen, die in der Prüfkammer 18 eingestellt sind und zu den Parametern des jeweiligen Analyseverfahrens gehören. Das Display 22 kann auch als Touchscreen ausgebildet werden und als Eingabemittel zur Steuerung des Systems 20 verwendet werden . Über dieses Display 22 können Parameter geändert, Parameter eingegeben oder die Messung gestartet bzw. beendet werden. Das Display 22 ist im Ausführungsbeispiel fest mit dem Gehäuse 17 verbunden, selbstverständlich besteht auch die Möglichkeit ein separates Display 22 zur Verfügung zu stellen, dass über die zuvor genannte Schnittstelle 21 mit dem System 20 verbunden wird. Das Display 22 kann selbstverständlich auch Teil einer Rechnereinheit sein, die ebenfalls im System 20 integriert oder mit dem System 20 verbindbar ausgebildet ist.

Das Gehäuse 17 weist im Ausführungsbeispiel zusätzlich ein Sichtfenster 23 auf, über das eine visuelle Inspektion der Proben möglich ist. Die visuelle Inspektion kann dabei mittels eines Mikroskops 24 oder einer Kameraeinrichtung 25, einem Fluoreszenzmikroskop oder eine Raman-Vorrichtung erfolgen, die jeweils mit dem System 20 verbunden, d.h. im Bereich des Gehäuses 17 oder auf oder in dem Gehäuse 17 angeordnet sind. In der Prüfkammer 18 selbst befindet sich im Ausführungsbeispiel gemäß Fig. 3 eine Beleuchtungseinheit 26, die als UV-Licht-Beleuchtungseinheit, Beleuchtungseinheit, welche ausgelegt ist, sichtbares Licht zu emittieren, Beleuchtungseinheit, welche ausgelegt ist, infrarotes Licht zu emittieren, Beleuchtungseinheit, welche ausgelegt ist, polarisiertes Licht zu emittieren, Beleuchtungseinheit, welche ausgelegt ist, fluoreszierendes Licht, insbesondere blaues, grünes oder rotes fluoreszierendes Licht, zu emittieren ausgebildet ist. Durch diese Beleuchtungseinheit 26 wird die visuelle Inspektion der Proben während der thermischen Analyse unterstützt.

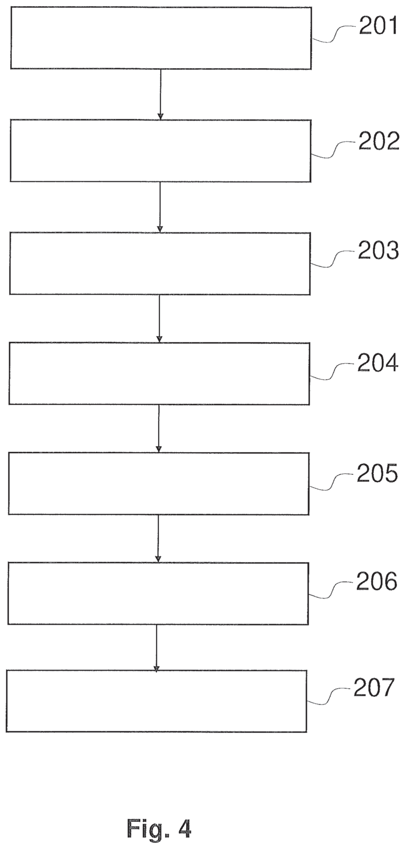

Fig. 4 zeigt ein Ablaufdiagramm eines Verfahrens zur simultanen Analyse einer Vielzahl von Einzelproben oder Gruppen von Einzelproben eines insbesondere biologischen Materials durch dynamische Differenzkalorimetrie (DSC), insbesondere in einem erfindungsgemäßen System wie zuvor beschrieben. Das Verfahren umfasst dabei die Schritte Einbringen 201 der Einzelproben in Probengefäße 11 in einem Probenträger 10, insbesondere einen Probenträger 10 wie zuvor beschreiben, mit dem jeweiligen Probegefäß 11 zugeordneten Einzelsensoren 12; Einbringen 202 des Probenträgers 10 in eine Heiz- und/oder Kühleinheit 19; Verbinden 203 der Einzelsensoren 12 mit einer Messeinrichtung; Durchführen 204 einer thermischen Analyse mit simultaner Messung von abgegebener oder aufgenommener Wärmemenge der Einzelproben während der thermischen Analyse durch die Einzelsensoren 12; Simultanes Erfassen 205 von Messwerten der Einzelproben oder Gruppen von Einzelproben durch die Messeinrichtung; Senden 206 der Messwerte an eine Auswerteeinheit 16, die mit der Messeinrichtung kommuniziert; und Simultanes Auswerten 207 der Messwerte und Ableitung charakterisierender Datenstrukturen der Einzelproben oder Gruppen von Einzelproben auf Basis der Messwerte mittels der Auswerteeinheit 16. Im erfindungsgemäßen Verfahren kann eine Analyse durch dynamische Differenzkalorimetrie (DSC), von insbesondere biologischem Material, beispielsweise Blut, Urin, Schweiß oder Hautgewebe tierischen oder menschlichen Ursprungs durchgeführt. Darüber hinaus können im Verfahren auch andere Materialien analysiert werden. Das Verfahren ist somit nicht auf die Verwendung mit biologischem Material beschränkt.

Zum Einbringen 201 der Einzelprobe wird diese in ein Probengefäß 11 eingelegt oder eingefüllt. Das Probengefäß 11 ist dabei Teil eines Probenträgers 10, der eine Vielzahl von Probengefäßen 11 umfasst. Bei diesem Probenträger 10 kann es sich beispielsweise um eine Mikrotiterplatte 14 mit standardisierter Konfiguration und Fläche handeln, das Einfüllen oder Einlegen der Probe kann beispielsweise durch Pipettieren erfolgen. Die Probe wird dabei auf die dem jeweiligen Probegefäß 11 zugeordneten Einzelsensoren 12 aufgebracht. Diese stehen jedoch nicht mit der Probe in unmittelbarer Berührung, sondern sind durch den Probenträger 10 von dieser getrennt. Gleichwohl ist der Probenträger 10 derart konfiguriert, dass eine verlustfreie Messung durch die Einzelsensoren 12 möglich ist.

Zum Einbringen 202 des Probenträgers 10 in eine Heiz- und/oder Kühleinheit 19 wird eine dort vorhandene Prüfkammer 18 geöffnet, und der Probenträger 10 dann in eine in der Prüfkammer 18 vorgesehene Aufnahme eingesetzt. Nach dem Einsetzen erfolgt das Verbinden 203 der Einzelsensoren 12 mit der Messeinrichtung. Dabei können die Einzelsensoren 12 jeweils einzeln mit der Messeinrichtung, beispielsweise über eine Steckverbindung, verbunden werden. Alternativ besteht auch die Möglichkeit, dass die jeweiligen Einzelsensoren 12 in einer Steckverbindung zusammengefasst sind, die dann mit einer entsprechenden Schnittstelle innerhalb der Prüfkammer 18 verbunden wird. In einer alternativen Ausführungsform sind die Einzelsensoren 12 in einer Sensorplatte 13 zusammengefasst, die zusätzlich die entsprechend zusammengefassten Leitungen der Einzelsensoren 12 aufweist und mit einem Steckverbinder zum Verbinden mit der Messeinrichtung ausgestattet ist.

Zum Durchführen der thermischen Analyse 204 erfolgt eine Temperaturbeaufschlagung des Probenträger 10 in der Prüfkammer 18. Die Beaufschlagung kann dabei mit einem definierten Temperaturprofil folgen oder bei konstanter Temperatur. Der Temperaturbereich kann dabei zwischen -200°C und +250°C, bevorzugt zwischen -80°C und +200°C liegen. Das System 20 wie auch der Probenträger 10 und die Einzelsensoren 12 bzw. die Sensorplatte 13 sind materialmäßig derart ausgeführt, dass die jeweils oberen wie auch unteren Temperaturbereiche zu keiner Beeinträchtigung der Messleistung bzw. Haltbarkeit der Elemente führt. Während der Temperaturbeaufschlagung wird durch die Einzelsensoren 12 die von den Einzelproben während der thermischen Analyse abgegebene oder aufgenommene Wärmemenge erfasst und als Sensorwert an eine Auswerteeinheit 16 weitergegeben. Im Verfahren ist dabei ein simultanes Erfassen 205 von Messwerten der Einzelproben oder Gruppen von Einzelproben durch die Messeinrichtung vorgesehen. D.h., dass in einem einzigen Messzyklus eine Vielzahl von Einzelproben verarbeitet werden kann und das Verfahren somit die Möglichkeit bietet den Probendurchsatz signifikant zu erhöhen. Bei Verwendung einer Mikrotiterplatte 14 mit beispielsweise 96 Probengefäßen können somit 96 Einzelproben gleichzeitig analysiert und die zugehörigen Messdaten entsprechend ausgegeben werden. Gegenüber herkömmlichen Verfahren mit Einzelmessung der Proben ergibt sich hieraus ein entscheidender Kapazitäts- und Zeitgewinn bei der Analyse. Die Konfiguration des Systems 20 stellt dabei sicher, dass verlässliche Einzelwerte für die jeweiligen Einzelproben erfasst und für die Analyse zur Verfügung gestellt werden. Die simultane Analyse erlaubt auch eine einzel- oder gruppenweise Auswertung von Messdaten für einzelne oder Gruppen von Einzelproben. Das Senden der Messwerte 206 an eine Auswerteeinheit 16, die mit der Messeinrichtung kommuniziert, erfolgt über eine Schnittstelle 21, die im System 20 vorgesehen ist. Das Senden kann dabei sowohl kabelgebunden wie auch kabellos, beispielsweise über eine Bluetooth- oder WLAN-Verbindung erfolgen. Die erfassten Messwerte werden dabei an eine nachgeordnete Auswerteinstanz, beispielsweise einen Computer, der mit entsprechender Software ausgestattet ist, weitergegeben und dort weiter analysiert. Gleichzeitig kann auch eine visuelle Darstellung der erfassten und oder ausgewerteten Messwerte auf einem in dem System 20 vorgesehenen Display 22 erfolgen, über das neben den Messergebnissen auch die Betriebsparameter des Systems 20, wie beispielsweise der Temperaturgradient in der Prüfkammer 18, dargestellt werden kann. Beim simultanen Auswerten 207 der Messwerte und der Ableitung von charakterisierenden Datenstrukturen der Einzelproben oder Gruppen von Einzelproben auf Basis der Messwerte werden die von den Einzelsensoren 12 zur Verfügung gestellten Rohdaten aufbereitet und für eine Detailauswertung der vorgenommenen Analyse zur Verfügung gestellt. Die Detailauswertung kann dabei in der Auswerteeinheit 16 oder in einer nachgeordneten Auswerteinstanz softwareunterstützt oder manuell durchgeführt werden.

In der vorangegangenen detaillierten Beschreibung sind verschiedene Merkmale zur Verbesserung der Stringenz der Darstellung in einem oder mehreren Beispielen zusammengefasst worden. Es sollte dabei jedoch klar sein, dass die obige Beschreibung lediglich illustrativer, keinesfalls jedoch beschränkender Natur ist. Sie dient der Abdeckung aller Alternativen, Modifikationen und Äquivalente der verschiedenen Merkmale und Ausführungsbeispiele. Viele andere Beispiele werden dem Fachmann aufgrund seiner fachlichen Kenntnisse in Anbetracht der obigen Beschreibung sofort und unmittelbar klar sein.

Die Ausführungsbeispiele wurden ausgewählt und beschrieben, um die der Erfindung zugrundeliegenden Prinzipien und ihre Anwendungsmöglichkeiten in der Praxis bestmöglich darstellen zu können. Dadurch können Fachleute die Erfindung und ihre verschiedenen Ausführungsbeispiele in Bezug auf den beabsichtigten Einsatzzweck optimal modifizieren und nutzen. In den Ansprüchen sowie der Beschreibung werden die Begriffe "beinhaltend" und "aufweisend" als neutralsprachliche Begrifflichkeiten für die entsprechenden Begriffe "umfassend" verwendet. Weiterhin soll eine Verwendung der Begriffe "ein", "einer" und "eine" eine Mehrzahl derartig beschriebener Merkmale und Komponenten nicht grundsätzlich ausschließen.

### Bezugszeichenliste

- 10: Probenträger
- 11: Probengefäß
- 12: Einzelsensor
- 13: Sensorplatte
- 14: Mikrotiterplatte
- 15: Boden
- 16: Auswerteeinheit
- 17: Gehäuse
- 18: Prüfkammer
- 19: Heiz- bzw. Kühleinheit
- 20: System
- 21: Schnittstelle
- 22: Display
- 23: Sichtfenster
- 24: Mikroskop
- 25: Kameraeinrichtung
- 26: Beleuchtungseinheit
- 201: Einbringen der Einzelprobe
- 202: Einbringen des Probenträgers
- 203: Verbinden der Einzelsensoren
- 204: Durchführen einer thermischen Analyse
- 205: Simultanes Erfassen von Messwerten
- 206: Senden der Messwerte
- 207: Simultanes Auswerten der Messwerte

- G: Grundfläche

## Patentansprüche

1. System (20) zur simultanen thermischen Analyse einer Vielzahl von Einzelproben eines, insbesondere biologischen, Materials durch dynamische Differenzkalorimetrie (DSC), mit:
- wenigstens einem, mehrere Probengefäße (11) aufweisenden Probenträger (10), wobei jedem Probengefäß (11) ein Einzelsensor (12) zur Messung einer von der Einzelprobe während der thermischen Analyse abgegebenen oder aufgenommenen Wärmemenge zugeordnet ist;
- einer Heiz- und/oder Kühleinheit (19) zur simultanen Temperaturbeaufschlagung der in den Probengefäßen (11) beinhalteten Einzelproben, mit einer Aufnahme für den wenigstens einen Probenträger (10);
- einer Messeinrichtung, welche mit den Einzelsensoren (12) verbunden ist, und dazu ausgebildet ist einen Messwert für die abgegebene oder aufgenommene Wärmemenge der Einzelproben während der thermischen Analyse simultan zu erfassen.

2. System (20) nach Anspruch 1, wobei die Aufnahme in einer Prüfkammer (18) angeordnet ist und wobei die Umgebungsbedingungen innerhalb der Prüfkammer (18) definiert oder variabel regelbar ausgebildet sind.

3. System (20) nach Anspruch 2, wobei die definierten oder variabel regelbaren Umgebungsbedingungen innerhalb der Prüfkammer (18) ausgewählt sind aus: Temperatur, Druck, relative Luftfeuchtigkeit, (Inert-) Gasatmosphäre und Kombinationen daraus.

4. System (20) nach einem der vorhergehenden Ansprüche, wobei ferner eine Auswerteeinheit (16) vorgesehen ist, welche dazu ausgebildet ist, die Messwerte von der Messeinrichtung zu empfangen, zu analysieren und charakterisierende Datenstrukturen auf Basis der Messwerte für jede Einzelprobe abzuschätzen.

5. System (20) nach einem der vorhergehenden Ansprüche, wobei ferner ein Display (22) vorgesehen ist, welches dazu ausgebildet ist, Messwerte und/oder Datenstrukturen zu visualisieren.

6. System (20) nach einem der vorhergehenden Ansprüche, wobei die Einzelsensoren (12) in einer Sensorplatte (13) zusammengefasst sind und die Anzahl der Einzelsensoren (12) der Sensorplatte (13) der Anzahl der Probengefäßen (11) im Probenträger (10) entspricht und wobei die Einzelsensoren (12) an Positionen der Probengefäße (11) entsprechenden Positionen in der Sensorplatte (13) angeordnet sind.

7. System (20) nach Anspruch 6, wobei die Sensorplatte (13) als gesondertes, mit dem Probenträger (10) form- oder kraftschlüssig verbindbares Element zur Verfügung gestellt ist.

8. System (20) nach Anspruch 6, wobei die Sensorplatte (13) als in der Aufnahme angeordnetes Element ausgebildet ist.

9. System (20) nach einem der Ansprüche 1 bis 5, wobei ein in jedes Probengefäß (11) integrierter oder ein mit jedem Probengefäß (11) form- oder kraftschlüssig verbindbarer Einzelsensor (12) vorgesehen ist.

10. System (20) nach einem der vorhergehenden Ansprüche, wobei eine Grundfläche des Einzelsensors (12) im Wesentlichen einer Grundfläche (G) des Probengefäßes (11) entspricht.

11. System (20) nach einem der vorhergehenden Ansprüche, wobei die Auswerteeinheit (16) ferner eine Schnittstelle (21) umfasst, welche dazu ausgebildet ist, eine Kommunikationsverbindung zwischen der Auswerteeinheit (16) und einem externen Kommunikationsteilnehmer aufzubauen.

12. System (20) nach Anspruch 11, wobei der externe Kommunikationsteilnehmer als Datenverarbeitungsgerät ausgebildet ist und über die Schnittstelle (21) mit der Auswerteeinheit (16) kommunikativ gekoppelt ist.

13. System (20) nach wenigstens einem der Ansprüche 1 bis 12, ferner umfassend eine Beleuchtungseinheit (26), wobei die Beleuchtungseinheit (26) ausgewählt ist aus: einer UV-Licht-Beleuchtungseinheit, einer Beleuchtungseinheit, welche ausgelegt ist, sichtbares Licht zu emittieren, einer Beleuchtungseinheit, welche ausgelegt ist, infrarotes Licht zu emittieren, einer Beleuchtungseinheit, welche ausgelegt ist, polarisiertes Licht zu emittieren, einer Beleuchtungseinheit, welche ausgelegt ist, fluoreszierendes Licht, insbesondere blaues, grünes oder rotes fluoreszierendes Licht, zu emittieren.

14. System (20) nach wenigstens einem der Ansprüche 1 bis 13, wobei die Heiz- und/oder Kühleinheit (19) , die Aufnahme für den wenigstens einen Probenträger, die Messeinrichtung, die Auswerteeinheit (16), die Prüfkammer (18), die Beleuchtungseinheit (26) und/oder das Display (22) von einem Gehäuse (17), insbesondere einem gemeinsamen Gehäuse (17), zumindest teilweise umgeben sind.

15. System (20) nach einem der vorhergehenden Ansprüche, wobei eine Schnittstelle (21) und/oder ein Sichtfenster (23) zur Anordnung einer optischen Auswertvorrichtung, insbesondere eine Kameravorrichtung (25), eine Mikroskopvorrichtung, ein Fluoreszenzmikroskop oder eine Raman-Vorrichtung vorgesehen ist.

16. System (20) nach Anspruch 14 oder 15, wobei die Schnittstelle (21) und/oder das Sichtfenster (23) in das Gehäuse (17) und/oder die Prüfkammer (18) integriert vorgesehen ist.

17. System (20) nach einem der vorhergehenden Ansprüche, wobei der Probenträger (10) als Mikrotiterplatte (14) mit standardisierter Konfiguration und die Aufnahme als Einsetzfach für die Mikrotiterplatte (14) ausgebildet ist.

18. Probenträger (10), insbesondere zur Verwendung in einem System (20) nach einem der vorhergehenden Ansprüche, wobei der Probenträger (10) eine Vielzahl von in einem definierten Raster angeordneten Probengefäßen (11) für jeweils eine Einzelprobe aufweist.

19. Probenträger (10) nach Anspruch 18, wobei der Probenträger (10) als aus einem temperaturbeständigen Material, insbesondere Kunststoffmaterial, gefertigte standardisierte Mikrotiterplatte (14) mit zwischen 6 und 1536 Probegefäßen (11) ausgebildet ist.

20. Probenträger (10) nach Anspruch 19, wobei das Kunststoffmaterial eine Temperaturbeständigkeit in einem Temperaturbereich von zwischen -200°C und +250°C, bevorzugt von zwischen -80°C und +200°C aufweist.

21. Probenträger (10) nach einem der Ansprüche 18 bis 20, wobei jedem Probengefäß (11) ein Einzelsensor (12) zur Messung von durch die Einzelprobe abgegebener oder aufgenommener Wärmemenge während einer thermischen Analyse zugeordnet ist.

22. Probenträger (10) nach Anspruch 21, wobei der Einzelsensor (12) in das Probengefäß (11) integriert oder mit dem Probengefäß (11) form- oder kraftschlüssig verbindbar ist.

23. Probenträger (10) nach einem der Ansprüche 18 bis 21, wobei ein der Anzahl an Probengefäßen (11) entsprechende Anzahl an Einzelsensoren (12) in einer mit dem Probenträger (10) form- oder kraftschlüssig verbindbaren Sensorplatte (13) zusammengefasst sind und die Einzelsensoren (12) an den Positionen der Probengefäße (11) entsprechende Positionen in der Sensorplatte (13) angeordnet sind.

24. Verfahren zur simultanen Analyse einer Vielzahl von Einzelproben oder Gruppen von Einzelproben eines insbesondere biologischen Materials durch dynamische Differenzkalorimetrie (DSC), insbesondere in einem System nach einem der Ansprüche 1 bis 17, umfassend:
- Einbringen (201) der Einzelproben in Probengefäße (11) in einen Probenträger (10), insbesondere einen Probenträger (10) nach einem der Ansprüche 18 bis 23, mit dem jeweiligen Probegefäß (11) zugeordneten Einzelsensoren (12);
- Einbringen (202) des Probenträgers (10) in eine Heiz- und/oder Kühleinheit;
- Verbinden (203) der Einzelsensoren (12) mit einer Messeinrichtung;
- Durchführen (204) einer thermischen Analyse mit simultaner Messung von abgegebener oder aufgenommener Wärmemenge der Einzelproben während der thermischen Analyse durch die Einzelsensoren (12);
- Simultanes Erfassen (205) von Messwerten der Einzelproben oder Gruppen von Einzelproben durch die Messeinrichtung;
- Senden der Messwerte (206) an eine Auswerteeinheit (16), die mit der Messeinrichtung kommuniziert; und
- Simultanes Auswerten (207) der Messwerte und Ableitung charakterisierender Datenstrukturen der Einzelproben oder Gruppen von Einzelproben auf Basis der Messwerte mittels der Auswerteeinheit (16).

25. Verfahren nach Anspruch 24, wobei die Analyse unter definierten oder variabel regelbaren Umgebungsbedingungen durchgeführt wird, wobei die definierten oder variabel regelbaren Umgebungsbedingungen ausgewählt sind aus: Temperatur, Druck, relative Luftfeuchtigkeit, (Inert-) Gasatmosphäre und Kombinationen daraus.

26. Verfahren nach einem der Ansprüche 24 oder 25, ferner umfassend eine visuelle Analyse der Einzelproben oder Gruppen von Einzelproben mittels einer optischen Auswertvorrichtung, insbesondere einer Kameravorrichtung (25), einer Mikroskopvorrichtung, einem Fluoreszenzmikroskop oder einer Raman-Vorrichtung.

27. Verfahren nach einem der Ansprüche 24 bis 26, wobei die Analyse in einem definierten Temperaturprofil durchgeführt wird.

28. Verfahren nach einem der Ansprüche 24 bis 27, wobei des simultane Auswerten (207) in der Auswerteeinheit (16) automatisiert oder manuell durchgeführt wird.

29. Verfahren nach wenigstens einem der Ansprüche 24 bis 26, ferner umfassend: Visualisieren der Datenstrukturen, insbesondere auf einem Display (22).

30. Verwendung eines Systems (20) nach wenigstens einem der Ansprüche 1 bis 17 und/oder eines Probeträgers (10) nach einem der Ansprüche 18 bis 23 zur simultanen thermischen und/oder visuellen Analyse von insbesondere biologischen Einzelproben durch dynamische Differenzkalorimetrie (DSC).
